(19) Europäisches Patentamt
European Patent Office
Office européen des brevets

(11) Publication number: **0 313 152 B1**

(12) # EUROPEAN PATENT SPECIFICATION

(45) Date of publication of patent specification: **24.03.93**

(51) Int. Cl.5: **C12N 11/14**, C12N 11/02

(21) Application number: **88202284.1**

(22) Date of filing: **13.10.88**

(54) **Process for the chemical immobilization of biologically active substances on silica.**

(30) Priority: **23.10.87 IT 2238987**

(43) Date of publication of application:
**26.04.89 Bulletin 89/17**

(45) Publication of the grant of the patent:
**24.03.93 Bulletin 93/12**

(84) Designated Contracting States:
**AT BE CH DE ES FR GB GR LI LU NL SE**

(56) References cited:
**EP-A- 0 207 564**
**EP-A- 0 291 130**
**US-A- 3 519 538**

(73) Proprietor: **ENIRICERCHE S.p.A.**
**Corso Venezia 16**
**I-20121 Milan(IT)**

(72) Inventor: **Battilotti, Massimo**
**Via Romolo Gigliozzi 53**
**I-00128 Tor de Cenci Rome(IT)**
Inventor: **Colapicchioni, Claudio**
**Via San Castulo 3**
**I-00182 Rome(IT)**
Inventor: **Fiorini, Mario**
**Viale Cortina d'Ampezzo 164**
**I-00135 Rome(IT)**
Inventor: **Barbaro, Aurelio**
**Via Madonna 2 Ponti**
**I-00060 Capena Rome(IT)**

(74) Representative: **Roggero, Sergio et al**
**Ing. Barzanò & Zanardo Milano S.p.A. Via Borgonuovo 10**
**I-20121 Milano (IT)**

EP 0 313 152 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to a process for chemically and stably immobilizing biologically active substances such as proteins (enzymes, antibodies, biological receptors, and so forth).

Such a process can be defined as economically advantageous and easy to achieve without the use of complex chemical reactions.

The use of enzymes (biocatalysts) immobilized on solid matrices is largely used for many industrial applications: e.g., for the production of intermediates, for preparing semi-synthetic penicillins, alimentary products and drugs in general.

The main advantage of the use of immobilized biocatalysts, both for continuous and batchwise processes, is that the catalyst can be re-used, with a consequent decrease in production costs.

A comprehensive survey about the immobilization of enzymes on solid supports is reported in "Trends in Biotechnology", vol. 3, 11, 1985, 276 Howard, H. Weetall.

In particular, as regards the chemical immobilization of biocatalysts on supports of inorganic type, such as silica, the process is carried out according to different routes, by using more or less complex chemical reactions.

One largely used from the available methods is the one based on the reaction of coupling, via a diazo-derivative obtained on a silica preliminarily treated with a suitable silane containing an amino-aromatic group.

Such a process is based on the formation of a diazo-linkage between the enzyme, which is a protein, and the aryl-diazo group present on the inorganic support, obtained after treatment with aminopropyl-triethoxy-silane and subsequent chemical treatments.

The reaction occurs thanks to the L-histidine and L-tyrosine moieties present in the protein. On the market, glass beads already exist, which are treated with organosilanes, and are traded by the Companies PIERCE CHEMICAL and ELECTRONUCLEONICS, both in diazo salt, and in nitro-group bearing form.

Another potential application for this type of reaction on silanized silica beads can be as an immuno-adsorbent.

In fact, the antibody or antigen, provided that is constituted by a protein immobilized via diazo-linkage on a silanized silica support, is used in the purification on column of the antigen or of the specific antibody, as reported by J. Immunological Methods 50, 57, 1982, Kennedy J. F., Keep P. A. and Catty D. Furthermore, the use of antibodies immobilized on silica could find some applications in the field of the immunodiagnostic tests.

In the prior art, the generally used route is illustrated, which provides for 3-aminopropyl-triethoxy-silane to be used as the silica-functionalising agent, and consists of a whole series of steps in order to come to the desired product, according to the following scheme:

1)

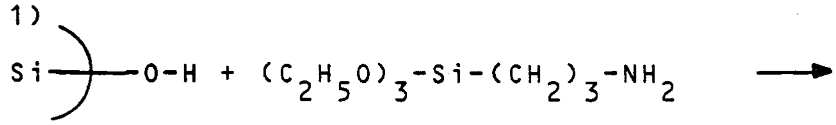

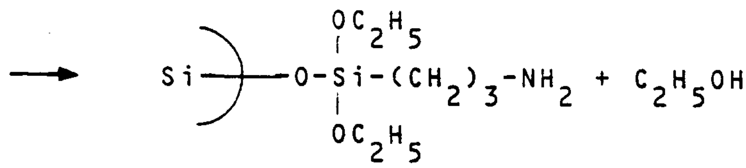

2)

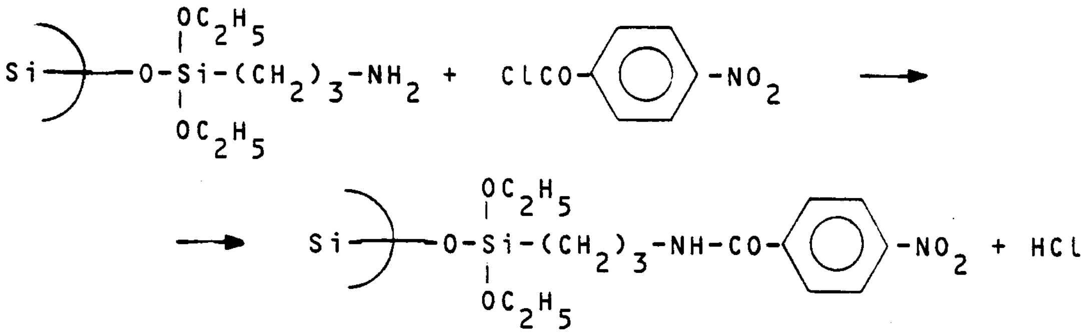

3)

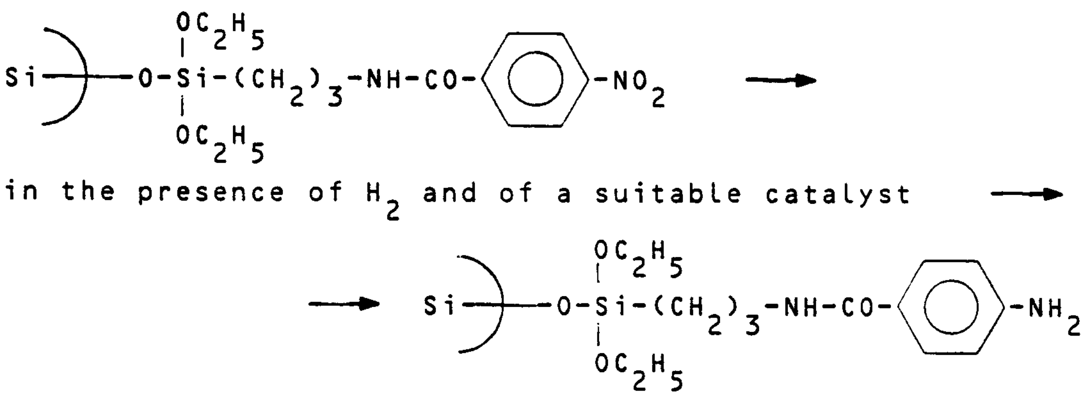


4)

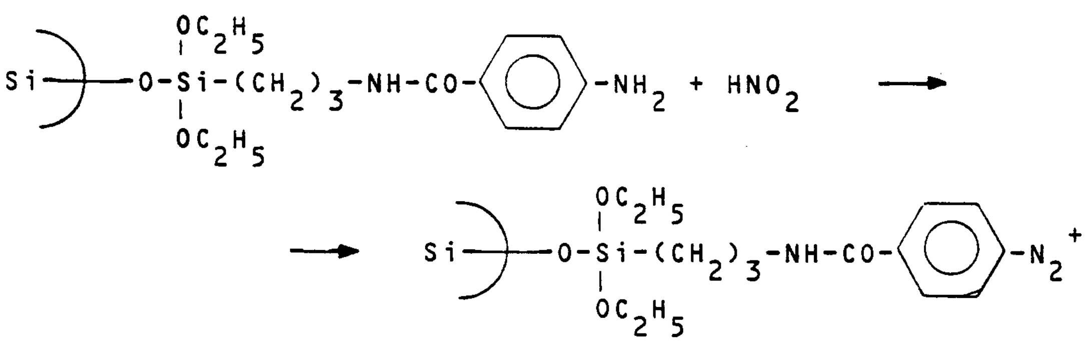

5)

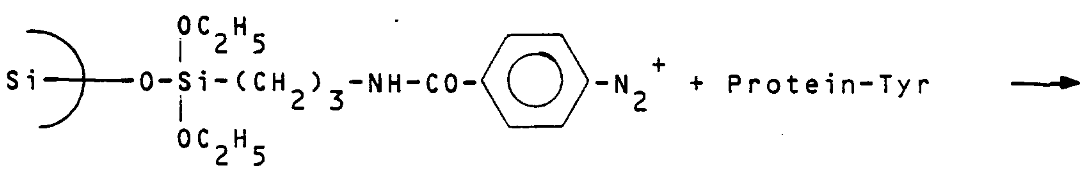

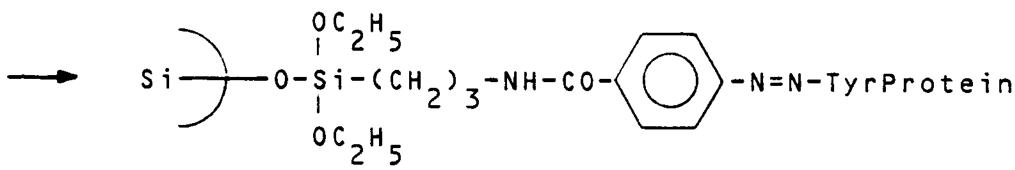

wherein:

Tyr = L-tyrosine residues in the protein.

As one can see from the above reaction scheme, in order to achieve the end product five steps are necessary, two of which, are delicate, and namely: the (2) step, wherein hydrochloric acid is released, which has to be removed from the reaction environment; and the (3) step, owing to the presence of a catalyst and the consequent complications inhering it its separation after the reaction, due to the fact that it must operate with solid-state reactants.

The possibility exists, that an aminophenyl-trimethoxy-silane may be used (as proposed by Mottola H.A., Analytical Chemistry 55, 2089, 1983): the evident advantage thereof is that it can be directly diazotised and coupled with the protein.

But, besides the considerably high cost of this product, the problems exist which arise from the considerable steric hyndrance due to the absence of a "spacer" between the silicon oxide moiety and the aromatic ring, and therefore the relative closeness of the amino group to the siliceous matrix.

To the above, the fact has to be added that the commercial product is constituted by a mixture of the three isomers. Other methods for activating silica via diazo compounds are reported on Talanta 32, 8B, 763, 1985 and Fres. Z. Anal. Chem. 322, 47, 1985, but anyway with very relative advantages.

EP-A-291 130 discloses a biosensor having an enzymatic membrane containing a layer of silicon oxide adhering to the membrane by means of a polysiloxanic matrix. It is therein excluded the use of a monomeric silane.

It has been surprisingly ascertained that, by using certain organosilanes, to be defined hereinafter, the immobilization of proteinous biologically active substances can be obtained with a few steps, higher yields than with the prior art procedures, and cheaply.

The invention, therefore, provides a process for chemically immobilizing on a silica support a proteinous biologically active substance by functionalizing said silica support with an organic silane, characterized in that it comprises the steps of:

1) Functionalizing the silica support with an exclusively monomeric organic silane selected from those having the general formula:

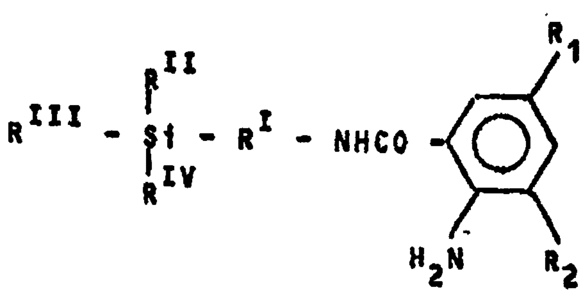

(I)

wherein:

$R_1$ and $R_2$, equal to, or different from one another, are selected from -Cl, -Br, $-CH_3$, $-NO_2$, -H and $-NH_2$;

$R^1$ is $(CH_2)_p$-X-$(CH_2)_q$, X is selected from $-CH_2$, monocondensed aromatic groups, polycondensed aromatic groups, =NH, and -O-, and p and q, equal to, or different from one another, are integers from 0 to 10, the 0 value being excluded when X is either =NH or is -O-;

$R^{II}$, $R^{III}$ and $R^{IV}$, equal to, or different from each other, are selected from the $C_1$-$C_{10}$ alkyl- and alkoxy groups;

2) Diazotizing the functionalized silica by preparing a diazo salt thereon, and

3) Contacting the proteinous biologically active substance concerned with the functionalized and diazotized silica to couple them chemically together.

The exclusively monomeric organosilanes of the general formula (I) are prepared, as disclosed, for example, in EP-A-86201103.8, or a derivative thereof, with an amino-silane of the general formula:

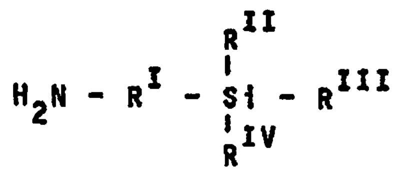

according to the following reaction scheme:

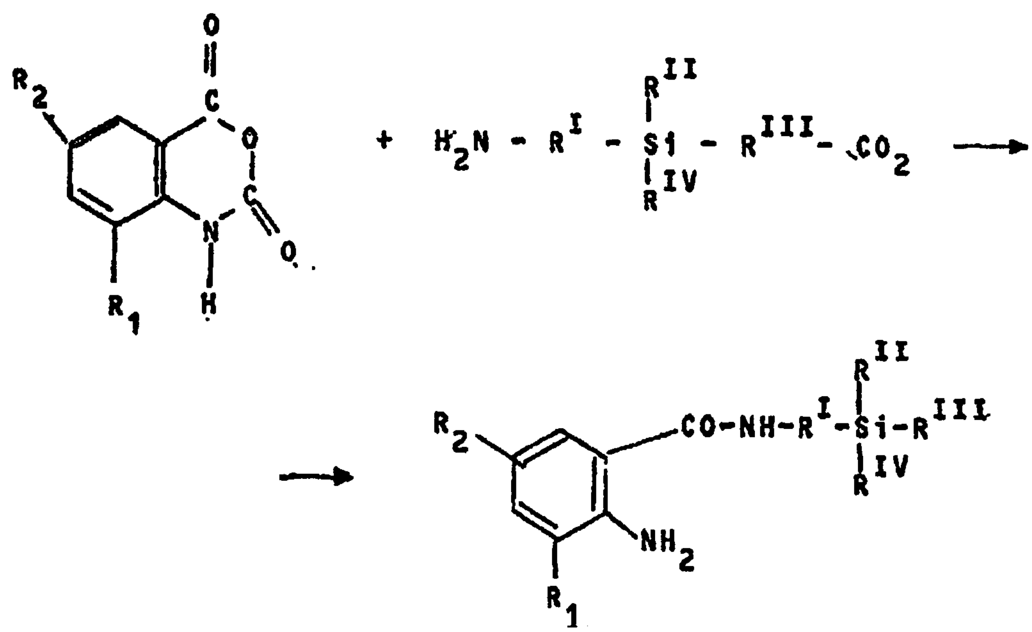

wherein $R_1$, $R_2$, $R^I$, $R^{II}$, $R^{III}$ and $R^{IV}$ have the same meanings as defined hereinabove.

The preferred aminosilanes are:

- 3-aminopropyl-triethoxy-silane
  $H_2N\text{-}(CH_2)_3\text{-}Si\text{-}(OC_2H_5)_3$,
- aminomethyl-triethoxysilane
  $H_2N\text{-}CH_2\text{-}Si\text{-}(OC_2H_5)_3$,
- N-(2-aminoethyl)-aminopropyl-trimethoxy-silane
  $H_2N\text{-}(CH_2)_2\text{-}NH\text{-}(CH_2)_3\text{-}Si\text{-}(OCH_3)_3$,
- N-(2-aminoethyl)-aminopropyl-triethoxy-silane
  $H_2N\text{-}(CH_2)_2\text{-}NH\text{-}(CH_2)_3\text{-}Si\text{-}(OC_2H_5)_3$,
- N-(2-aminoethyl)-aminopropyl-dimethoxy-methyl-silane

$$H_2N\text{-}(CH_2)_2\text{-}NH\text{-}(CH_2)_3\text{-}\underset{\substack{|\\ CH_3}}{Si}\text{-}(OCH_3)_2$$

The reaction of synthesis is carried out by gradually adding isatoic anhydride to the amino-silane, in the presence or in the absence of a solvent.

The solvents are alcohols, ethers, chlorinated solvents and are water-free.

The reaction is carried out under room pressure and at room temperature.

The functionalisation of the silica with the organo-silanes of general formula (I) can be accomplished by placing the same silica into contact with the selected organo-silane under reaction conditions well-known to those skilled in the art.

Once that the functionalised silica is prepared, and the coupling-capable biologically active substance is selected, the functionalised silica is diazotised.

A second method for functionalising silica consists in placing silica into contact with aminopropyl-triethoxy-silane (AAPS) and then carrying out the reaction with isatoic anhydride under heterogeneous phase conditions.

The diazotisation is carried out with nitrous acid in a known way, at a temperature comprised within the range of from 0 ° C to 25 ° C in water-alcoholic solution (methyl-, ethyl-, propyl alcohol-water solution), at an acidic pH value.

The coupling between the functionalised and diazotised silica and the biologically active substance is carried out by placing into intimate contact the two species, and operating at a temperature comprised within the range of from o ° C to 25 ° C, and at a pH comprised within the range of from 5 to 8.

By considering 2-amino-N(3-triethoxy-silyl-propyl)benzamide (anthranylamido-propyl-triethoxy-silane) of formula:

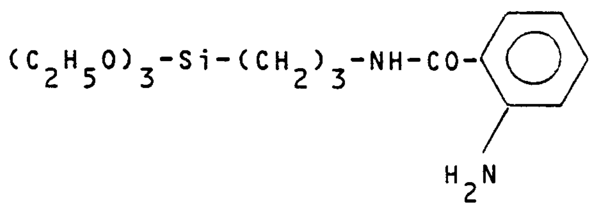

as an exemplifying functionalising agent for the silica, and, as the substance, an enzyme or an antibody, the reaction scheme is as follows:

1)

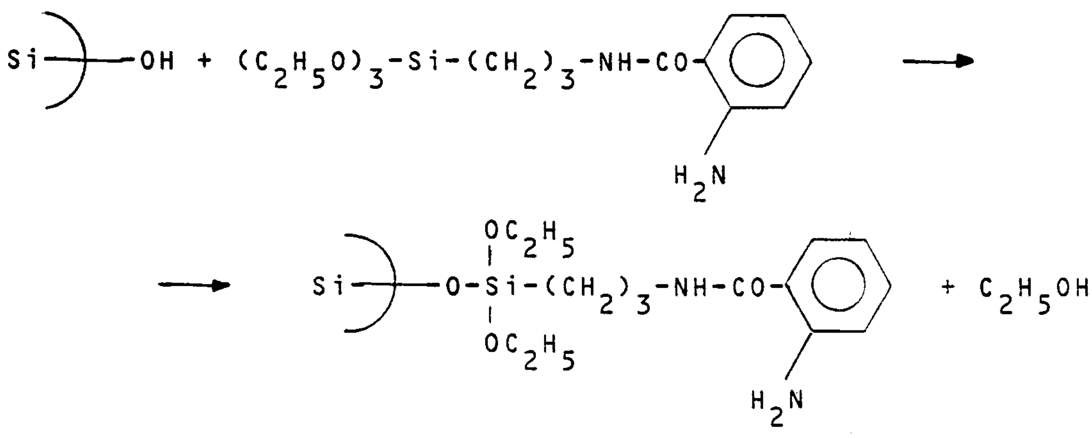

2)

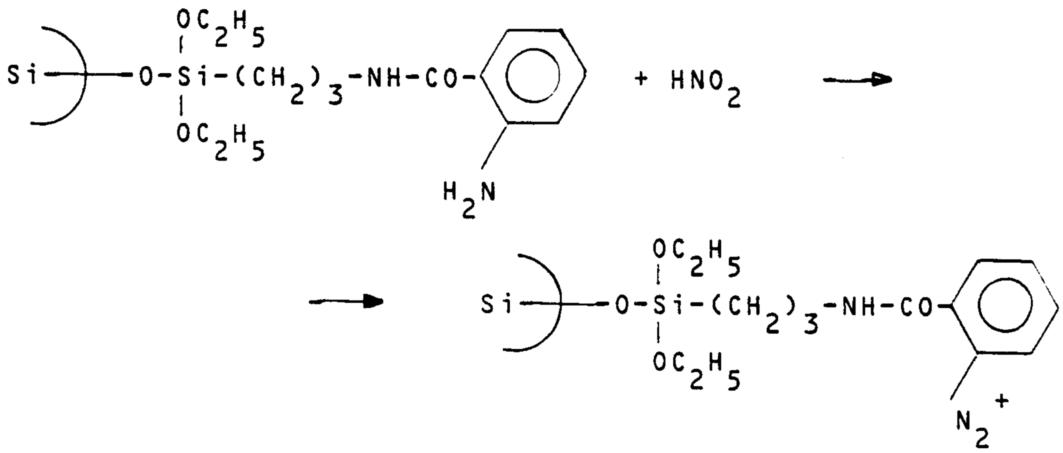

3)

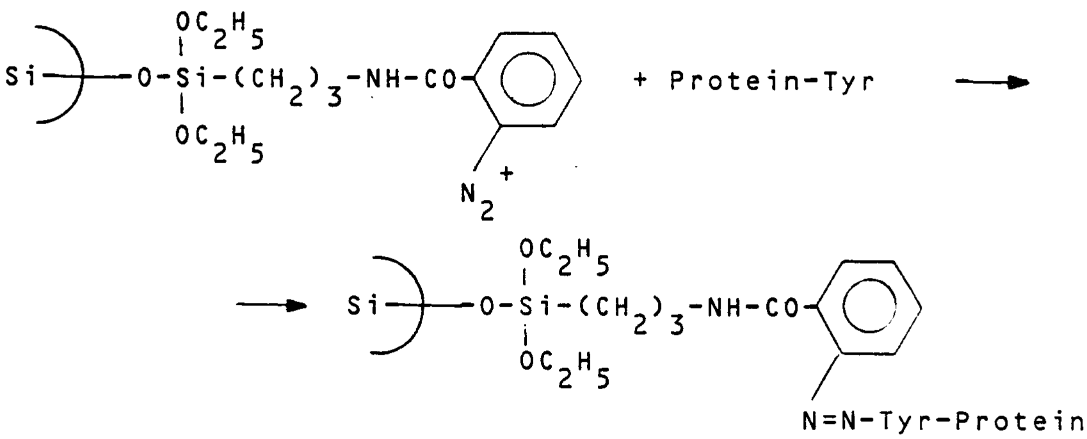

wherein:

Tyr = L-Tyrosine residues in the protein.

The fact that, notwithstanding the $-NH_2$ group is in the ortho-position relatively to the carbonyl in the group

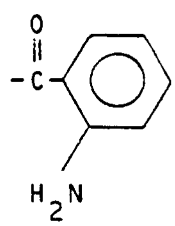

the $-NH_2$ function, as its diazo salt, is reactive towards the biological active substance, is quite surprising.

The above disclosed process of chemical immobilization can be also carried out on other types of solid inorganic supports, such as, e.g., supports of $TiO_2$, $SnO_2$, and so forth.

Some examples are now given, which have the purpose of better illustrating the present invention, it being understood that the same invention has not to be considered as being limited by them or to the.

Example 1

Trypsin Immobilization

a) Reaction of silica with silane

2 g of $SiO_2$ (Merck 60, 70-230 mesh, 0.063-0.200 mm) is treated with 2.0 ml of anthranylamide-propyl-triethoxy-silane (AAPS) in 20 ml of anhydrous toluene.

The reaction system is maintained 16 hours under refluxing condition sunder vacuum, with mechanical stirring. The treated silica is filtered, and washed a plurality of times with hot toluene, and finally with absolute ethanol. The silica charged to the oven in order to be dried weighed 2.4 g.

b) Diazo salt formation

To a small jacketed reactor, 1 g of $SiO_2$ treated with silane is charged, and to it 15 ml of 2 N HCl is added. With mechanical stirring, and at the temperature of 5°C, 5 l of $H_2O$ containing 1 g of $NaNO_2$ is added dropwise.

The overall reaction time is of 2 hours. The reaction product is subsequently repeatedly washed on a porous septum with 0.1 M phosphate buffer at pH 7.

c) Immobilization of trypsin via diazo-coupling

To a small jacketed reactor, 1 g of treated $SiO_2$ as a diazo salt, 10 ml of 50 mM phosphate buffer at pH 7.5, 20 mg of trypsin (Boehringer Biochemia Robin) and 0.015 M $CaCl_2$ are charged.

The solution is kept with slow mechanical stirring at the temperature of 5°C for about 2 hours.

The silica is subsequently washed on a porous septum with buffer solution.

The treated and washed silica is stored in 0.046 M Tris buffer at pH 8.1 at 5°C, 50 mM $CaCl_2$.

Hydrolytic Activity of the Trypsin in a Closed-Loop Flow-System and in an Open-Loop Flow System

The system inside which the measurement tests are carried out is constituted by a peristaltic pump, a spectrophotometer containing a temperature-controlled cuvette, and a small reactor inside which the enzyme immobilized on silica is present.

The whole system is connected to a series of rubber hoses as shown in Figure 1, wherein:

A = substrate;
W = drain;
P = peristaltic pump;
V = valves for closed/open-loop system;
R = small reactor containing $SiO_2$ + linked enzyme;
S = spectrophotometer;
C = temperature-controlled cuvette;
E = estinction.

The solution which contains the substrate is constituted by 46 mM Tris buffer pH 8.1, 100 mg of $CaCl_2$, 0.5 mM, 1 mM, 2 mM benzoyl-L-arginine-ethyl-ester (BAEE).

The hydrolytic activity is checked by measuring the extinction at 254 nm of the solution after the passage of the immobilized enzyme.

In Figure 2, the reaction kinetics are plotted as a function of the concentration of the substrate in a closed-loop-flow system (a: BAEE = 2 mM - T = 25°C; b: BAEE = 1 mM - T = 25°C; c: BAEE = 0.5 mM - T = 25°C).

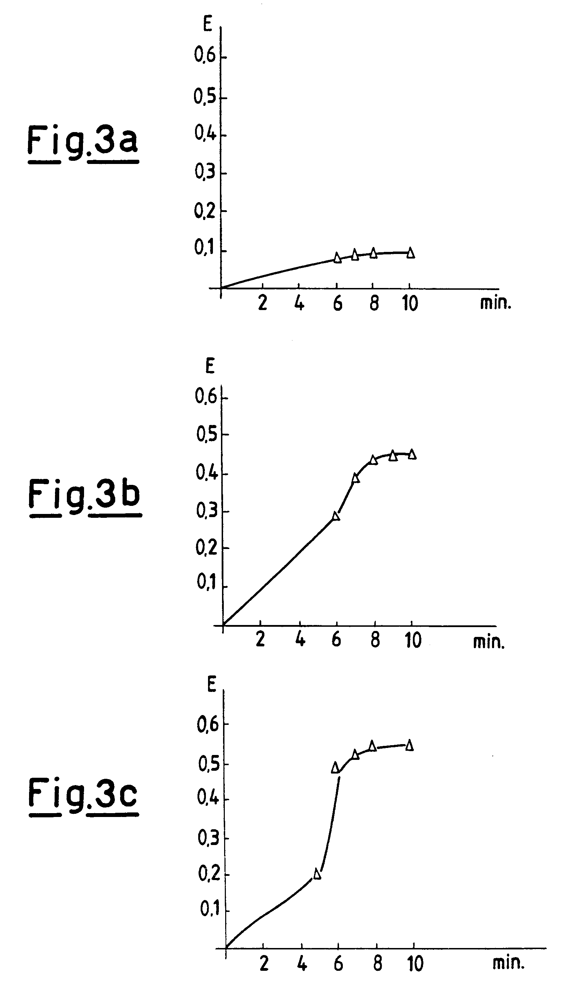

In Figure 3, the same reaction kinetics are reported for an open-loop-flow system (a: BAEE = 2 mM - T = 25°C; b: BAEE = 1 mM - T = 25°C; c: BAEE = 0.5 mM - T = 25°C).

In Figure 4, the values of maximum activity of the enzyme (closed-loop system) are reported, wherein the activity is expressed as the extinction values as a function of substrate concentration. A good linearity can be observed, even if in the presence of a possible inhibition of the substrate.

Example 2

Immobilization of Chymotrypsin

The (a) and (b) steps are the same as of Example 1.

e) Immobilization of chymotrypsin via diazo-coupling.

To a small jacketed reactor, 1 g of treated $SiO_2$ as a diazo salt, 10 ml of 0.1 M phosphate buffer at pH 7.4, 2 mg of indole, 25 mg of chymotrypsin (Boehringer Biochemia Robin) are added.

The solution is maintained with slow mechanical stirring, and at the temperature of 5°C for about 2 hours.

Finally, the silica is washed a plurality of times with buffer solution on a porous septum.

The silica is stored in 0.1 M Tris buffer at pH 7.8, 50 mM $CaCl_2$, at 5°C.

In Table 1, the amounts of the residual aminoacids are reported as % by weight, after treatment of the enzyme-containing silica in 6 M HCl at 100°C for 18 hours.

The values were obtained with an automatic aminoacid analyser.

Example 3

Immobilization of Thermolysin

The (a) and (b) steps are the same as of Example 1.

e) Immobilization of thermolysin via diazo-coupling.

To a small jacketed reactor, 1 g of treated $SiO_2$ as a diazo salt, and a solution containing 15 ml of phosphate buffer at pH 7.5, 30 mg of thermolysin (Agrade, Lot 100540, Calbiochem, Calif.), 10 mM $CaCl_2$ are added.

The solution is maintained with slow mechanical stirring at the temperature of 5 °C for about 2 hours. The silica is finally washed with buffer solution on a porous septum.

The silica is stored in Tris buffer - 50 m HCl at 5 °C.

In Table 2, the amounts of the residual aminoacids are reported as % by weight, after treatment in 6 N HCl at 100 °C for 18 hours of the enzyme-containing silica.

The values were obtained with an automatic aminoacid analyser.

TABLE 1

Chymotrypsin Immobilized on $SiO_2$

| AMINOACIDS | mg/100 mg |
|---|---|
| Lysine | 0.022 |
| Histidine | 0.0068 |
| Arginine | 0.0054 |
| Aspartic acid | 0.039 |
| Treonine | 0.015 |
| Serine | 0.046 |
| Glutamic acid | 0.030 |
| Glycine | 0.025 |

TABLE 1 (cont.d)

Chymotrypsin Immobilized on $SiO_2$

| AMINOACIDS | mg/100 mg |
|---|---|
| Alanine | 0.016 |
| Cysteine | 0.018 |
| Valine | 0.016 |
| Methionine | 0.0031 |
| Isoleucine | 0.016 |
| Leucine | 0.023 |
| Tyrosine | 0.019 |
| Phenyl-alanine | 0.0071 |
| TOTAL | 0.3074 |

TABLE 2

Thermolysin Immobilized on $SiO_2$

| AMINOACIDS | mg/100 mg |
|---|---|
| Lysine | 0.032 |
| Histidine | 0.016 |
| Arginine | 0.024 |
| Aspartic acid | 0.102 |
| Treonine | 0.047 |
| Serine | 0.045 |
| Glutamic acid | 0.055 |
| Glycine | 0.045 |
| Alanine | 0.042 |
| Cysteine | - |
| Valine | 0.037 |
| Methionine | - |
| Isoleucine | 0.032 |
| Leucine | 0.035 |
| Tyrosine | 0.082 |
| Phenyl-alanine | 0.033 |

TABLE 2 (cont.d)

Thermolysin Immobilized on $SiO_2$

| AMINOACIDS | mg/100 mg |
|---|---|
| TOTAL | 0.627 |

## Claims

1. Process for chemically immobilizing on a silica support a proteinous biologically active substance by functionalizing said silica support with an organic silane, characterized in that it comprises the steps of:
1) Functionalizing the silica support with an exclusively monomeric organic silane selected from those having the general formula:

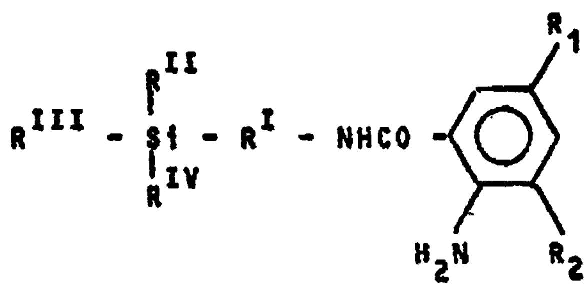

(I)

wherein:
$R_1$ and $R_2$, equal to, or different from one another, are selected from -Cl, -Br, $-CH_3$, $-NO_2$, -H and $-NH_2$;
$R^1$ is $(CH_2)_p$-X-$(CH_2)_q$, X is selected from $-CH_2$, monocondensed aromatic groups, polycondensed aromatic groups, =NH, and -O-, and p and q, equal to, or different from one another, are integers from 0 to 10, the 0 value being excluded when X is either =NH or is -O-;
$R^{II}$, $R^{III}$ and $R^{IV}$, equal to, or different from each other, are selected from the $C_1$-$C_{10}$ alkyl- and alkoxy groups;
2) Diazotizing the functionalized silica by preparing a diazo salt thereon, and
3) Contacting the proteinous biologically active substance concerned with the functionalized and diazotized silica to couple them chemically together.

2. Process according to Claim 1, wherein step 2) is carried out with nitrous acid, at a temperature of from 0 °C to 25 °C, in a water-alcoholic solution at an acidic pH.

3. Process according to Claim 1, wherein step 3) is carried out at a temperature of from 0 °C to 25 °C and at a pH of from 5 to 8.

4. Process according to Claim 1, wherein said exclusively monomeric organic silane is the product of the reaction of the isatoic acid anhydride, or a derivative thereof, with:
3-aminopropyl-triethoxy-silane, of formula:

$H_2N$-$(CH_2)_3$-Si-$(OC_2H_5)_3$

5. Process according to Claim 1, wherein said exclusively monomeric organic silane is the product of the reaction of the isatoic acid anhydride, or a derivative thereof, with:
aminomethyl-triethoxysilane, of formula:

$H_2N$-$CH_2$-Si-$(OC_2H_5)_3$

**6.** Process according to Claim 1, wherein said exclusively monomeric organic silane is the product of the reaction of the isatoic acid anhydride, or a derivative thereof, with: (N-(2-aminoethyl)-aminopropyl-trimethoxy silane) of formula:

$H_2N-(CH_2)_2-NH-(CH_2)_3-Si-(OCH_3)_3$

**7.** Process according to Claim 1, wherein said exclusively monomeric organic silane is the product of the reaction of the isatoic acid anhydride, or a derivative thereof, with: (N-(2-aminoethyl)-aminopropyl-triethoxy silane) of formula:

$H_2N-(CH_2)_2-NH-(CH_2)_3-Si-(OC_2H_5)_3$

**8.** Process according to Claim 1, wherein said exclusively monomeric organic silane is the product of the reaction of the isatoic acid anhydride, or a derivative thereof, with: (N-(2-aminoethyl)-aminopropyl-dimethoxy methyl silane) of formula:

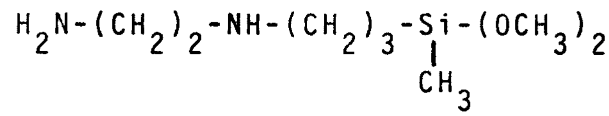

**9.** Process according to Claim 1, wherein step 1) is carried out by contacting the silica with the selected exclusively monomeric organic silane.

**10.** Process according to Claim 1, wherein step 1) is carried out by first contacting the silica with aminopropyl-triethoxy-silane, and reacting the resultant product with the isatoic acid anhydride under heterogeneous phase conditions thereafter.

## Patentansprüche

**1.** Verfahren zum chemischen Immobilisieren einer biologisch wirksamen Proteinsubstanz auf einem Siliziumdioxidträger durch Funktionalisieren dieses Siliziumdioxidträgers mit einem organischen Silan, dadurch gekennzeichnet, daß es die folgenden Stufen umfaßt:

1) Funktionalisieren des Siliziumdioxidträgers mit einem ausschließlich monomeren organischen Silan, ausgewählt unter jenen mit der allgemeinen Formel:

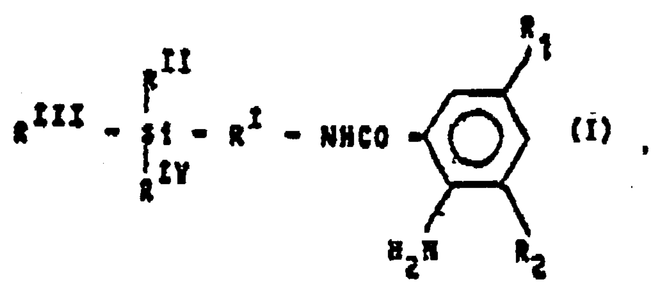

worin:

$R_1$ und $R_2$, die gleich oder voneinander verschieden sind, unter -Cl, -Br, $-CH_3$, $-NO_2$, -H und $-NH_2$ ausgewählt sind;

$R^I$ für $(CH_2)_p$-X-$(CH_2)_q$ steht, X unter $-CH_2$, monokondensierten aromatischen Gruppen, polykondensierten aromatischen Gruppen, -NH und -O-ausgewählt ist und p und q, die gleich oder voneinander verschieden sind, ganze Zahlen von 0 bis 10 bedeuten, wobei der Wert 0 ausgeschlossen ist, wenn X entweder für -NH oder für -O- steht;

$R^{II}$, $R^{III}$ und $R^{IV}$, die gleich oder voneinander verschieden sind unter $C_1$-$C_{10}$-Alkyl- und -Alkoxygruppen ausgewählt sind;

2) Diazotieren des funktionalisierten Siliziumdioxids durch Herstellen eines Diazosalzes; und
3) Kontaktieren der entsprechenden, biologisch aktiven Proteinsubstanz mit dem funktionalisierten und diazotierten Siliziumdioxid, um sie chemisch aneinander zu kuppeln.

**2.** Verfahren nach Anspruch 1, worin die Stufe 2) bei einer Temperatur von 0 ° C bis 25 ° C in einer wäßrig-alkoholischen Lösung bei einem sauren pH-Wert mit Salpetriger Säure ausgeführt wird.

**3.** Verfahren nach Anspruch 1, worin die Stufe 3) bei einer Temperatur von 0 ° C bis 25 ° C und bei einem pH-Wert von 5 bis 8 ausgeführt wird.

**4.** Verfahren nach Anspruch 1, worin das Ausschließlich monomere organische Silan das Reaktionsprodukt von Isatosäureanhydrid oder einem Derivat hievon mit 3-Aminopropyl-triethoxy-silan der Formel:

$H_2N-(CH_2)_3-Si-(OC_2H_5)_3$

ist.

**5.** Verfahren nach Anspruch 1, worin das ausschließlich monomere organische Silan das Reaktionsprodukt von Isatosäureanhydrid oder einem Derivat hievon mit Aminomethyl-triethoxysilan der Formel:

$H_2N-CH_2-Si-(OC_2H_5)_3$

ist.

**6.** Verfahren nach Anspruch 1, worin das auschließlich monomere organische Silan das Reaktionsprodukt von Isatosäureanhydrid oder einem Derivat hievon mit N-(2-Aminoethyl)-aminopropyl-trimethoxysilan der Formel:

$H_2N-(CH_2)_2-NH-(CH_2)_3-Si-(OCH_3)_3$

ist.

**7.** Verfahren nach Anspruch 1, worin das ausschließlich monomere organische Silan das Reaktionsprodukt von Isatosäureanhydrid oder einem Derivat hievon mit N-(2-Aminoethyl)-aminopropyl-triethoxysilan der Formel:

$H_2N-(CH_2)_2-NH-(CH_2)_3-Si-(OC_2H_5)_3$

ist.

**8.** Verfahren nach Anspruch 1, worin das ausschließlich monomere organische Silan das Reaktionsprodukt von Isatosäureanhydrid oder einem Derivat hievon mit N-(2-Aminoethyl)-aminopropyl-dimethoxy-methylsilan der Formel:

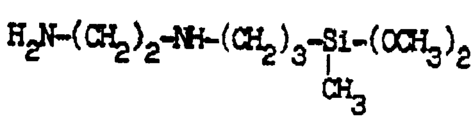

ist.

**9.** Verfahren nach Anspruch 1, worin die Stufe 1) durch Kontaktieren des Siliziumdioxids mit dem ausgewählten ausschließlich monomeren organischen Silan ausgeführt wird.

**10.** Verfahren nach Anspruch 1, worin die Stufe 1) dadurch ausgeführt wird, daß zunächst das Siliziumdioxid mit Aminopropyl-triethoxy-silan kontaktiert wird und anschließend des gebildete Produkt mit dem Isatosäureanhydrid unter Bedingungen der heterogenen Phase umgesetzt wird.

**Revendications**

1. Procédé pour immobiliser chimiquement, sur un support en silice, une substance protéinique biologiquement active, par fonctionnalisation dudit support en silice avec un silane organique, caractérisé en ce qu'il comporte les étapes de :
   1) fonctionnalisation du support en silice, avec un silane organique exclusivement monomère, choisi parmi ceux qui présentent la formule générale :

   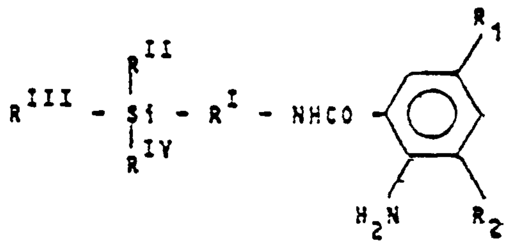

   (I)

   dans laquelle $R_1$ et $R_2$, identiques ou différents l'un de l'autre, sont choisis parmi -Cl, -Br, $-CH_3$, $-NO_2$, -H et $-NH_2$ ;
   $R^I$ représente $-(CH_2)_p-X-(CH_2)_q-$, X étant choisi parmi $-CH_2-$, les groupes aromatiques monocondensés, les groupes aromatiques polycondensés, -NH- et -O-, et p et q, égaux ou non, représentant des nombres entiers valant de 0 à 10, la valeur 0 étant exclue quand X représente -NH- ou -O- ;
   $R^{II}$, $R^{III}$ et $R^{IV}$, identiques ou différents l'un de l'autre, sont choisis parmi les groupes alkyle et alkoxy en $C_{1-10}$ ;
   2) diazotation de la silice fonctionnalisée, par préparation d'un sel de diazonium de celle-ci ; et
   3) mise en contact de la substance protéinique biologiquement active concernée et de la silice fonctionnalisée et diazotée, afin de les coupler ensemble chimiquement.

2. Procédé selon la revendication 1, dans lequel l'étape (2) est effectuée à l'aide d'acide nitreux, à une température de 0 ° C à 25 ° C, en solution aqueuse-alcoolique et à pH acide.

3. Procédé selon la revendication 1, dans lequel l'étape (3) est effectuée à une température de 0 ° C à 25 ° C et à un pH de 5 à 8.

4. Procédé selon la revendication 1, dans lequel ledit silane organique exclusivement monomère est le produit de la réaction de l'anhydride d'acide isatoïque, ou de l'un de ses dérivés, avec le 3-aminopropyl-triéthoxy-silane de formule:

   $H_2N-(CH_2)_3-Si(OC_2H_5)_3$

5. Procédé selon la revendication 1, dans lequel ledit silane organique exclusivement monomère est le produit de la réaction de l'anhydride d'acide isatoïque, ou de l'un de ses dérivés, avec l'aminométhyl-triéthoxy-silane de formule:

   $H_2N-CH_2-Si(OC_2H_5)_3$

6. Procédé selon la revendication 1, dans lequel ledit silane organique exclusivement monomère est le produit de la réaction de l'anhydride d'acide isatoïque, ou de l'un de ses dérivés, avec le N-(2-aminoéthyl)-aminopropyl-triméthoxy-silane de formule:

   $H_2N-(CH_2)_2-NH-(CH_2)_3-Si(OCH_3)_3$

7. Procédé selon la revendication 1, dans lequel ledit silane organique exclusivement monomère est le produit de la réaction de l'anhydride d'acide isatoïque, ou de l'un de ses dérivés, avec le N-(2-aminoéthyl)-aminopropyl-triéthoxy-silane de formule:

   $H_2N-(CH_2)_2-NH-(CH_2)_3-Si(OC_2H_5)_3$

**8.** Procédé selon la revendication 1, dans lequel ledit silane organique exclusivement monomère est le produit de la réaction de l'anhydride d'acide isatoïque, ou de l'un de ses dérivés, avec le N-(2-aminoéthyl)-aminopropyl-diméthoxy-méthylsilane de formule:

$H_2N\text{-}(CH_2)_2\text{-}NH\text{-}(CH_2)_3\text{-}Si(CH_3)(OCH_3)_2$

**9.** Procédé selon la revendication 1, dans lequel on effectue l'étape (1) en mettant la silice en contact avec le silane organique exclusivement monomère qu'on a choisi.

**10.** Procédé selon la revendication 1, dans lequel on effectue l'étape (1) en mettant d'abord la silice en contact avec de l'aminopropyl-triéthoxy-silane, et en faisant ensuite réagir le produit résultant avec de l'anhydride d'acide isatoïque, en phase hétérogène.

Fig.1

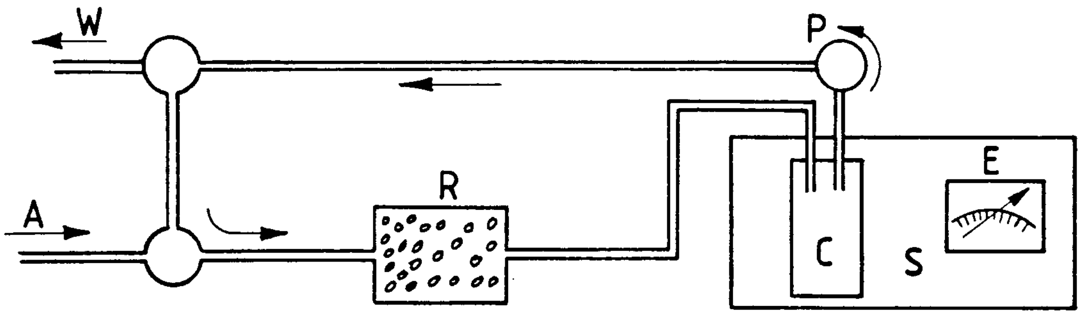


Fig.4

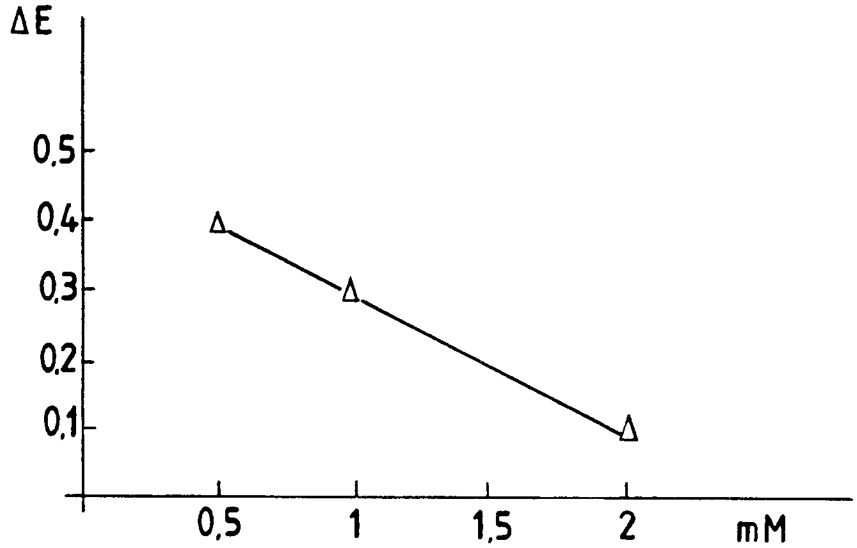

Fig.2a

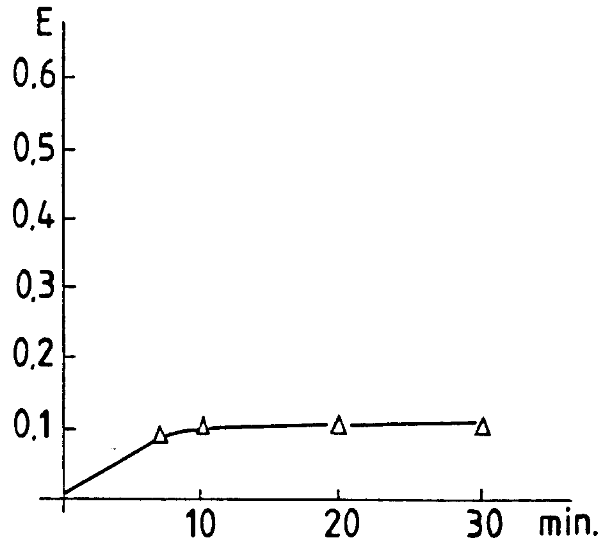


Fig.2b

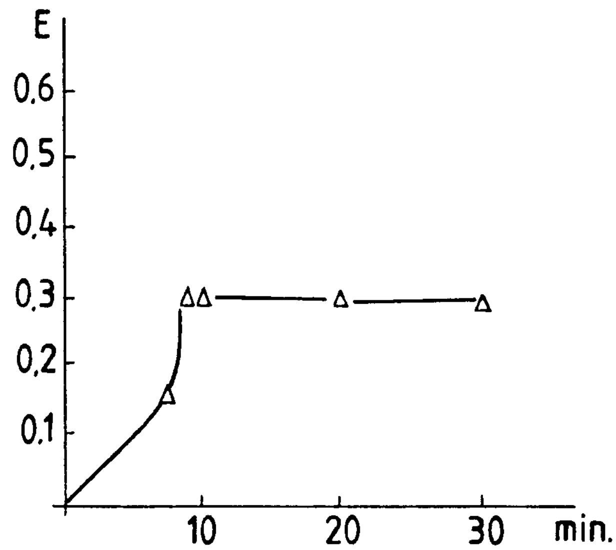


Fig.2c

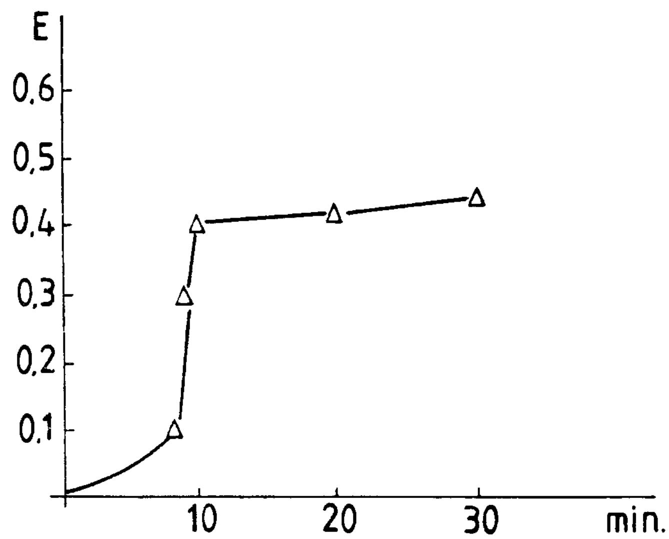

Fig.3a

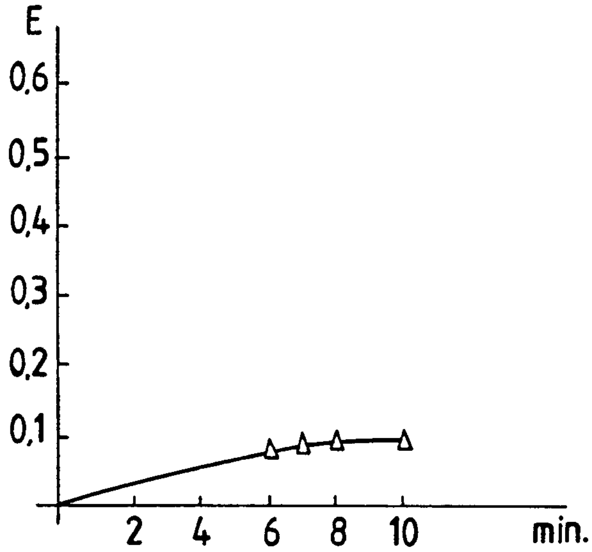


Fig.3b

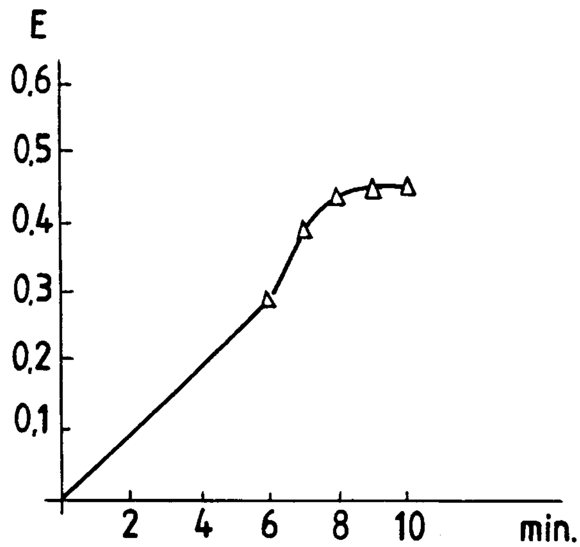


Fig.3c